Europäisches Patentamt

European Patent Office

Office européen des brevets

(11) Veröffentlichungsnummer : **0 023 311**
**B2**

(12) **NEUE EUROPÄISCHE PATENTSCHRIFT**

(45) Veröffentlichungstag der neuen Patentschrift :
**10.02.88**

(21) Anmeldenummer : **80104127.8**

(22) Anmeldetag : **16.07.80**

(51) Int. Cl.⁴ : **A 61 N 5/06**, A 61 N 5/08,
H 01 J 61/02

(54) **Medizinisches Bestrahlungsgerät.**

(30) Priorität : **27.07.79 DE 2930458**

(43) Veröffentlichungstag der Anmeldung :
**04.02.81 Patentblatt 81/05**

(45) Bekanntmachung des Hinweises auf die Patenterteilung : **09.02.83 Patentblatt 83/06**

(45) Bekanntmachung des Hinweises auf die Entscheidung
über den Einspruch : **10.02.88 Patentblatt 88/06**

(84) Benannte Vertragsstaaten :
**AT BE CH FR GB IT LI LU NL SE**

(56) Entgegenhaltungen :
**EP-B- 0 023 311
DE-A- 2 438 164
DE-A- 2 537 855
DE-A- 2 559 610
DE-A- 2 652 480
DE-A- 2 705 264
GB-A- 1 186 366
GB-A- 1 468 556
JP-A-74 067 892
US-A- 2 198 770
US-A- 3 431 215
US-A- 3 658 068
US-A- 3 986 513
"Fluorescent Lamp Phosphors' von Butler, The Pennsylvania State University Press, 1980, S. 16-18
Zeitschrift "Photochemistry and Photobiology",
1976, Vol. 24, S. 613-615
Chemical Abstracts, Vol. 82, 1975, 37922 w
P. Bocionek et al., Zum Einfluss von UV-Bestrahlung
auf das Tumorwachstum, Zeitschrift für physikalische
Medizin, Demeter Verlag, Gräfelfing (1982), S. 217-219
"Strahlentherapie", Zeitschrift f. Radiologie und
Onkologie; Sonderdruck, Urban & Schwarzenberg
"Philips-Prospekt"**

(73) Patentinhaber : **Wolff, Friedrich**
**Störklingasse 38**
**CH-4125 Riehen/Basel (CH)**

(72) Erfinder : **Wolff, Friedrich**
**Störklingasse 38**
**CH-4125 Riehen/Basel (CH)**

(74) Vertreter : **Knoblauch, Ulrich, Dr.-Ing.**
**Kühhornshofweg 10**
**D-6000 Frankfurt am Main 1 (DE)**

## Beschreibung

Die Erfindung bezieht sich auf ein medizinisches Bestrahlungsgerät zur Behandlung von karzinomatös verändertem Zellgewebe.

Es ist ein Bestrahlungsgerät zur Behandlung der Psoriasis (Schuppenflechte) bekannt, mit dessen Hilfe die an der unteren Grenze der Keimschicht der Haut befindlichen Erreger dieser Krankheit durch UVA-Strahlung zum Absterben gebracht werden sollen (US-A-39 86 513). Hierbei liegt der Patient auf einer strahlungsdurchlässigen Liegeplatte. Er wird von oben und unten und wenigstens einer Seite mit Hilfs von UV-Lampen bestrahlt. Als für diesen Zweck geeignete Lampen sind Philips-Leuchtstofflampen TL/05 und TL/09 bekannt. Beim älteren Typ lag das Maximum des Emissionsspektrums bei 365 nm und beim neueren Typ bei 360 nm. Da hierbei die Haut durch medikamentöse Behandlung, nämlich die Verabreichung von Melanin, sensibilisiert werden muß, ist man in neuerer Zeit bestrebt, die Strahlung in den Bereich der energiereicheren kürzeren Wellen zu verschieben (DE-A-27 07 920).

Es ist ferner bekannt, UVA-Bestrahlungsgeräte zu Bräunungszwecken einzusetzen. Hierbei ist für eine Pigmentdunkelung eine Strahlung unter 350 nm und für eine Pigmentbildung eine Strahlung unter 340 nm erforderlich.

Des weiteren sind medizinische Bestrahlungsgeräte bekannt, die IR-Strahlung abgeben, hauptsächlich im kurzwelligen IR-Bereich Solche mit Glühkörpern (Glühlampen, Quarzstäben o dgl.) ausgestatteten Geräte sollen die Blutzirkulation anregen und dadurch die Heilung, beispielsweise einer Entzündung, beschleunigen.

Darüber hinaus ist es bekannt, in einem Bestrahlungsgerät eine UV-Strahlungsquelle, z. B. eine Quecksilber-Hochdrucklampe, und eine IR-Strahlungsquelle, z. B. einen Quarzstab, anzuordnen.

Unter vielen anderen ist ein Leuchtstoff bekannt, der Strontiumfluorborat, aktiviert mit Europium, aufweist (US-PS 3 431 215). Das Maximum des Emissionsspektrums liegt bei etwa 370 nm. Die 50 %-Bandbreite des Emissionsspektrums ist kleiner als 30 nm. Ein Anwendungszweck ist nicht angegeben.

Der Erfindung liegt die Aufgabe zugrunde, ein zur Behandlung von karzinogen verändertem Zellgewebe besonders geeignetes medizinisches Bestrahlungsgerät anzugeben.

Diese Aufgabe wird erfindungsgemäß gelöst durch eine Strahlungsquelle, deren Emissionsspektrum, d. h. Energieverteilung über Wellenlänge, im UVA-Bereich, d. h. 315-400 nm, ein Maximum hat, wobei das Maximum des Emissionsspektrums zwischen 367 und 385 nm liegt und die 50 %-Bandbreite des Emissionsspektrums nicht größer als 30 nm ist, und durch ein Filter, das Strahlung unter 340 nm in wesentlichen abfiltert.

Vorzugsweise liegt das Maximum zwischen 370 und 380 nm und insbesondere zwischen 370 und 372 nm.

Ganz überraschend wurde festgestellt, daß durch eine Bestrahlung mit diesem Gerät durch Karzinogene krankhaft verändertes Zellgewebe regeneriert wird. Dieser Effekt tritt nicht nur bei Entzündungen o. dgl. an der Hauptoberfläche oder dicht darunter auf, sondern läßt sich mittels einer Oberflächenbestrahlung auch im Körperinneren erzielen. Dieser Erfolg wird mit einer UV-Strahlung erzielt, die im bisher unbeachteten, sehr langwelligen Teil des UVA-Bereichs liegt. Die Wellenlänge von etwa 380 nm scheint eine Anregungsfrequenz darzustellen, die bestimmte Selbstheilungskräfte in Gang setzt. Um hier eine ausreichende UV-Energie zur Verfügung zu stellen, das Maximum in der Nähe dieser Wellenlänge liegen.

Da beim Durchtritt durch die Haut oder vorgehaltenes Zellgewebe Transmissionsverluste auftreten : die eine Verschiebung ins Langwelligere bewirken, werden Maxima unter 380 nm bevorzugt.

Günstig ist es, wenn das Emissionsspektrum zum kurzwelligen Bereich so stark abfällt, daß die relative Energie bei 350 nm weniger als 20 % des Maximums beträgt. Hierdurch wird die UVA-Strahlungsenergie auf den gewünschten Bereich konzentriert. Darüber hinaus wird Pigmentbildung, Pigmentdunkelung und Erythembildung um so mehr vermindert, je kleiner der Strahlungsanteil unter 350 nm ist. Auf diese Weise wird die Strahlungsdurchlässigkeit der Haut nicht beeinträchtigt und die Applikationszeit nicht begrenzt.

Diese Effekte können noch verbessert werden durch ein Filter, das Strahlung unter 350 nm im wesentlichen abfiltriert.

Um eine möglichst gezielte, energiesparende Beeinflussung zu erreichen, sollte die 50 %-Bandbreite des Emissionsspektrums vorzugsweise nur etwa 20 nm betragen.

Bei einem bevorzugten Ausführungsbeispiel ist dafür gesorgt, daß die Strahlungsquelle außerdem IR-Strahlung im Bereich zwischen 6 000 und 9 000 nm emittiert. Für die Abgabe dieser IR-Strahlung kann auch eine zusätzliche Strahlungsquelle vorgesehen sein. Diese langwellige IR-Strahlung entspricht einer sehr milden Temperatur, etwa im Bereich der Körpertemperatur. Überraschenderweise läßt sich durch die Kombination dieser IR-Strahlung mit der beanspruchten UVA-Strahlung die Behandlung des erkrankten Zellgewebes noch rascher zum Erfolg führen.

Außerdem wird die Tiefenwirkung der Bestrahlung damit verbessert.

Zweckmäßigerweise liegt im Bereich zwischen 6 000 und 9 000 nm mindestens 30 %, vorzugsweise mindestens 50 % der gesamten IR-Strahlung vor. Die Energie soll daher auf die tatsächlich wirksamen Spektralbereiche konzentriert werden.

Es gibt zahlreiche Möglichkeiten, die UV Strahlung im gewünschten Bereich zu erzeugen. Besonders günstig ist jedoch eine Hg-Niederdruck-Leuchtstofflampe, weil es durch entsprechende Wahl des Leuchtstoffes gelingt, den größten Teil der abgestrahlten UVA-Energie im gewünschten Bereich anzuordnen. Beispielsweise kann der Leuchtstoff Strontiumfluorborat und Europium aufweisen. Ein weiterer Vorteil liegt darin, daß eine solche Leuchtstofflampe derart betrieben werden kann, daß die IR-Strahlung im Bereich zwischen 6 000 und 9 000 nm abgibt.

Mit Vorteil ist die Strahlungsquelle stabförmig und von einem rinnenförmigen Reflektor umgeben. Der Reflektor erhöht die Strahlungsdichte vor dem Gerät.

Einen großen Vorteil bietet es, wenn mehrere stabförmige Strahlungsquellen dicht benachbart nebeneinander und mit geringem Abstand von der Strahlenaustrittsöffnung des Geräts angeordnet sind. Dies ergibt ein Gerät für die flächige Bestrahlung von Körperpartien. Bei der Verwendung von 5 bis 20 stabförmigen Strahlungsquellen mit einer Länge von 1,50 oder 1,80 m lassen sich auch Ganzkörperbestrahlungen durchführen.

Da von den Strahlungserzeugern nur eine milde Wärme oder gar keine Wärme verlangt wird, kann der Patient sehr dicht an die UVA-Strahlungsquelle herangeführt werden, wo sich eine entsprechend hohe Strahlungsdichte einstellt. Günstig sind daher auch sämtliche Merkmale, die es gestatten, den Patienten dicht vor den Strahlungserzeugern zu plazieren. Bei einer bevorzugten Ausführungsform ist dafür gesorgt, daß die in einer etwa horizontalen Ebene angeordneten Strahlungsquellen von einer etwa horizontalen, zumindest für UV-Strahlung über 350 nm durchlässigen Liegeplatte in geringem Abstand überdeckt sind. Hierbei ergibt sich automatisch ein geringer Abstand zwischen Patient und Strahlungsquellen.

Eine andere Möglichkeit besteht darin, daß die in einer etwa horizontalen Ebene angeordneten Strahlungsquellen in einem Gehäuse mit nach unten gerichteter Strahlenaustrittsöffnung untergebracht sind, wobei das Gehäuse mittels Motor und zugehöriger Steuerung auf und ab bewegbar ist. Der Patient kann eine Bestrahlungsliege bequem besteigen und dann kann er oder ein Dritter das Gehäuse bis dicht über seinen Körper absenken.

Die Erfindung wird nachstehend anhand in der Zeichnung dargestellter, bevorzugter Ausführungsbeispiele näher erläutert. Es zeigt

Fig. 1 das Emissionsspektrum im UV-Bereich einer erfindungsgemäß verwendeten Strahlungsquelle,

Fig. 2 das Emissionsspektrum im IR-Bereich einer erfindungsgemäß verwendeten Strahlungsquelle,

Fig. 3 einen schematischen Querschnitt durch ein Bestrahlungsgerät gemäß der Erfindung,

Fig. 4 eine schematische Seitenansicht einer anderen Ausführungsform eines Bestrahlungsgeräts,

Fig. 5 die Vorderansicht einer weiteren Ausführungsform eines Bestrahlungsgeräts und

Fig. 6 einen Querschnitt durch eine als Leuchtstofflampe ausgebildete Strahlungsquelle.

UV-Strahlung wird unterteilt in UVC-Strahlung bis 280 nm, UVB-Strahlung zwischen 280 und 315 nm und UVA-Strahlung zwischen 315 und 400 nm. Anschließend folgt das sichtbare Licht bis etwa 750 nm. Dann schließt sich der IR-Bereich bis 10 000 nm an.

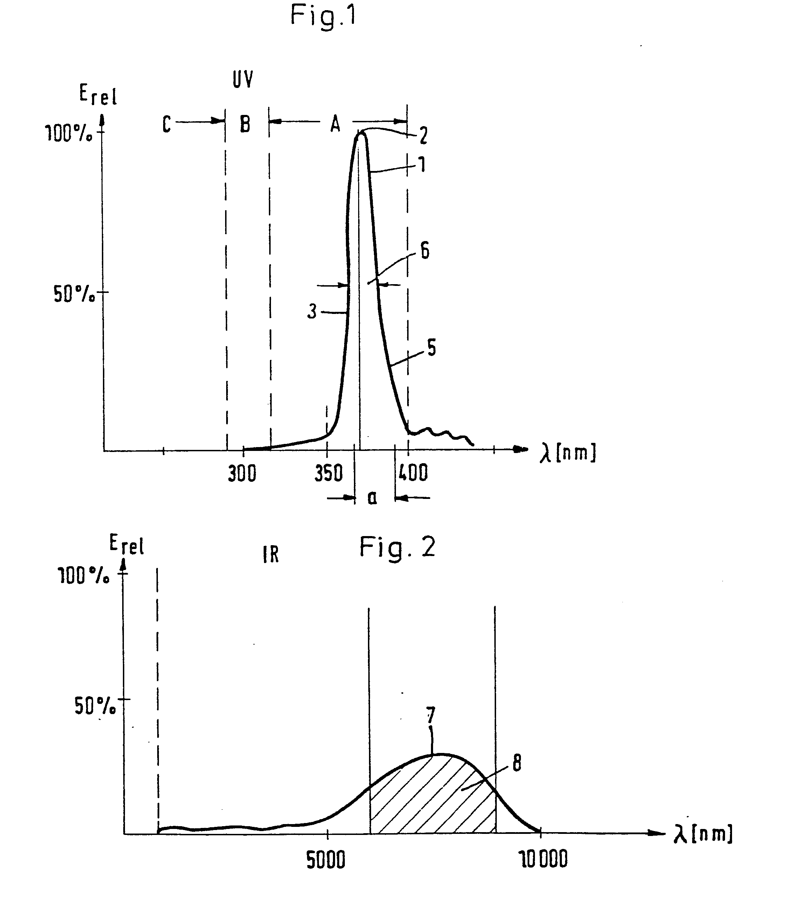

In der Fig. 1 und 2 ist für ein bevorzugtes Ausführungsbeispiel angegeben, wie das Emissionsspektrum bei einem erfindungsgemäßen Bestrahlungsgerät ausgebildet sein kann. Hierzu ist über der Wellenlänge λ die relative Energie $E_{rat}$ aufgetragen. Hier zeigt sich, daß eine ausgeprägte Energiespitze 1 im langwelligen Teil der UVA-Strahlung vorgesehen ist. Das Maximum 2 dieser Energiespitze 1 liegt im Bereich a, der von 367 bis 385 nm reicht, vorzugsweise aber durch 370 und 380 nm begrenzt ist. Hier liegt das Maximum 2 bei 371 nm. Die dem kurzwelligen Teil der UVA-Strahlung zugewandte Flanke 3 der Spitze 1 fällt steil ab, so daß unterhalb 350 nm nur noch eine geringe Strahlungsenergie vorhanden ist. Gegebenenfalls kann ein Filter verwendet werden, das die Strahlung unterhalb dieser Wellenlänge im wesentlichen abfiltert. Pigmentfärbung, Pigmentbildung und Erythembildung werden daher weitgehend verhindert. Auch die Flanke 5 der Spitze 1 fällt verhältnismäßig steil ab, so daß oberhalb 400 nm nur noch geringe Energie abgestrahlt wird. Insgesamt ergibt sich daher eine Spitze 1, die eine 50 %-Bandbreite 6 von 19 nm hat. Nahezu die gesamte Abstrahlung im UVA-Bereich ist daher auf die Strahlung zwischen 360 und 390 nm konzentriert.

Während die Strahlung im Bereich des sichtbaren Lichts und im kurzwelligen Teil der IR-Strahlung vergleichsweise gering ist, ergibt sich im langwelligen Teil der IR-Strahlung ein weiteres Maximum 7 im Emissionsspektrum, wobei der Hauptteil im schraffierten Bereich 8, also zwischen 6 000 und 9 000 nm abgestrahlt wird.

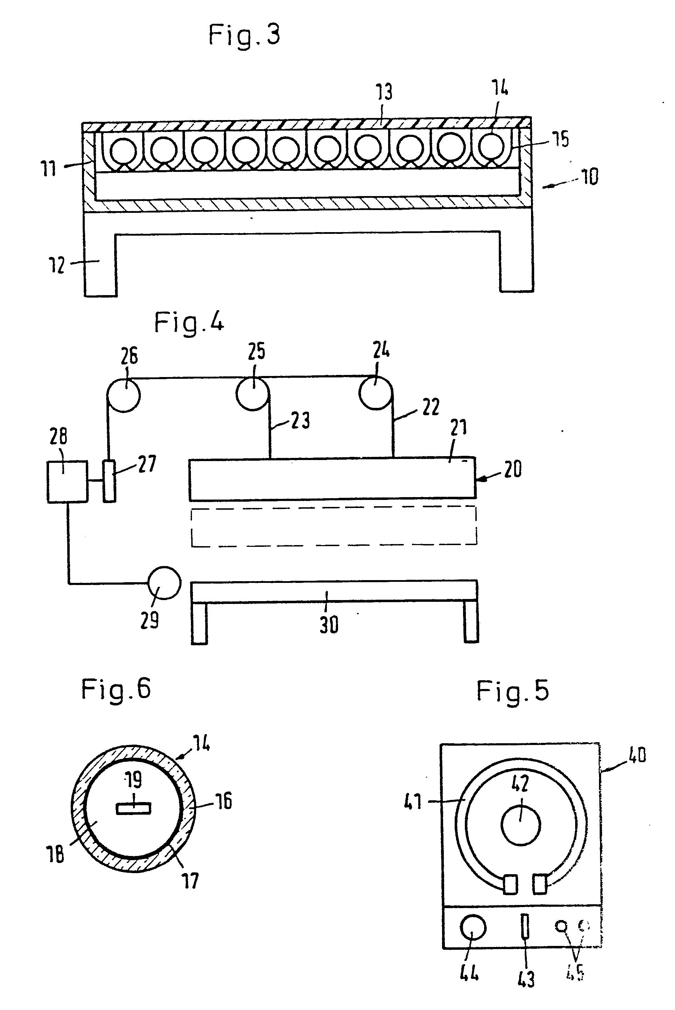

In Fig. 3 ist eine Ausführungsform eines erfindungsgemäßen Bestrahlungsgeräts veranschaulicht. Es handelt sich um eine Liege 10, bei der ein Gehäuse 11 auf einem Fußgestell 12 ruht und oben von einer Liegeplatte 13 überdeckt ist, die zumindest für die hier interessierende Strahlung durchlässig ist.

Im Gehäuse sind Hg-Niederdruck-Leuchtstofflampen 14 je in einem rinnenförmigen Reflektor 15 derart angeordnet, daß die Strahlung nach oben abgegeben wird. Die Leuchtstoffröhren sind so dicht benachbart, daß ihr Abstand kleiner als ihr Durchmesser ist. Auch ihr Abstand zur Unterseite der Liegeplatte 13 ist kleiner als ihr Durchmesser. Die Liegeplatte 13 kann auch als Filter dienen, beispielsweise, um Strahlung unterhalb 340 oder 350 nm abzufiltern. Bei der Behandlung legt sich der Patient auf die Liegeplatte 13, so daß sein ganzer Körper von der Strahlung aus der Spitze 1 und dem Bereich 8 getroffen wird.

In Fig. 4 ist ein Bestrahlungsgerät 20 gezeigt, dessen Gehäuse 21 eine ähnliche Innenausstattung wie das Gehäuse 10 hat, aber eine nach unten gerichtete Strahlenaustrittsöffnung aufweist. Das Gehäuse hängt an Seilen 22 und 23, die über Umlenkrollen 24, 25 und 26 zu einer Wickelvorrichtung 27 geführt sind, welche von einem Motor 28 antreibbar ist. Dieser weist eine Fernsteuervorrichtung 29 auf. Das Gehäuse ist über eine Liege 30 angeordnet und kann mit Hilfe des Motors 28 aus der mit durchgehenden Linien gezeichneten Stellung, in der ein Patient die Liege besteigen kann, durch Betätigen der Fernsteuervorrichtung 29 in die gestrichelt gezeichnete Stellung abgesenkt werden, in der sich die Strahlenaustrittsöffnung dicht über dem Patienten befindet.

Bei der Ausführungsform nach Fig. 5 ist ein Bestrahlungsgerät 40 gezeigt, das eine kreisförmige gebogene Leuchtstoffrohr 41 als UVA-Strahlung abgebende Quelle und in der Mitte eine zusätzliche Strahlungsquelle 42 aufweist, die IR-Strahlung im langwelligen Bereich abgibt, beispielsweise eine schwach beheizte Metallplatte. Außerdem sind an diesem Gerät ein Ein- und Ausschalter 43, eine Zeitschaltuhr 44 und Anzeigelampen 45 angebracht. Derartige Teile können auch in den Geräten 10 und 20 untergebracht sein.

Fig. 6 zeigt eine Hg-Niederdruck-Leuchtstofflampe 14 im Querschnitt. Ein Glasmantel 16, der gleichzeitig als Filter für Strahlung unterhalb 350 nm dienen kann, trägt an der Innenseite eine Leuchtstoffschicht 17, welche Strontiumfluoroborat und Europium aufweist. Der Innenraum 18 ist mit Quecksilberdampf unterhalb des atmosphärischen Drucks gefüllt. An den stirnseitigen Enden befinden sich Elektroden 19. Eine solche Lampe vermag ein Spektrum entsprechend Fig. 1 abzugeben. Durch Wahl der Vorschaltgeräte hat man es in der Hand, die Lampe mit einer solchen Leistung zu betreiben, daß auch die gewünschte IR-Strahlung entsprechend Fig. 2 abgegeben wird.

## Patentansprüche

1. Medizinisches Bestrahlungsgerät zur Behandlung von karzinomatös verändertem Zellgewebe, gekennzeichnet durch eine Strahlungsquelle, deren Emissionsspektrum, d. h. Energieverteilung über Wellenlänge, im UVA-Bereich, d. h. 315-400 nm, ein Maximum hat, wobei das Maximum des Emissionsspektrums zwischen 367 und 385 nm liegt und die 50 %-Bandbreite des Emissionsspektrums nicht größer als 30 nm ist, und durch ein Filter (4, 13, 16), das Strahlung unter 340 nm im wesentlichen abfiltert.

2. Bestrahlungsgerät nach Anspruch 1, dadurch gekennzeichnet, daß das Maximum zwischen 370 und 380 nm liegt.

3. Bestrahlungsgerät nach einem der Ansprüche 1 oder 2, dadurch gekennzeichnet, daß das Maximum zwischen 370 und 372 nm liegt.

4. Bestrahlungsgerät nach Anspruch 1 bis 3, dadurch gekennzeichnet, daß das Emissionsspektrum zum kurzwelligen Bereich so stark abfällt, daß die relative Energie bei 350 nm weniger als 20 % des Maximums beträgt.

5. Bestrahlungsgerät nach einem der Ansprüche 1 bis 4, dadurch gekennzeichnet, daß das Filter (4, 13, 16) Strahlung unter 350 nm im wesentlichen abfiltert.

6. Bestrahlungsgerät nach Anspruch 5, dadurch gekennzeichnet, daß die 50 %-Bandbreite des Emissionsspektrums etwa 20 nm beträgt.

7. Bestrahlungsgerät nach einem der Ansprüche 1 bis 6, dadurch gekennzeichnet, daß die Strahlungsquelle (14) außerdem IR-Strahlung im Bereich zwischen 6 000 und 9 000 nm emittiert.

8. Bestrahlungsgerät nach einem der Ansprüche 1 bis 6, dadurch gekennzeichnet, daß eine zusätzliche Strahlungsquelle (42) vorgesehen ist, die IR-Strahlung im Bereich zwischen 6 000 und 9 000 nm emittiert.

9. Bestrahlungsgerät nach Anspruch 7 oder 8, dadurch gekennzeichnet, daß im Bereich zwischen 6 000 und 9 000 nm mindestens 30 % der gesamten IR-Strahlung vorliegt.

10. Bestrahlungsgerät nach Anspruch 9, dadurch gekennzeichnet, daß im Bereich zwischen 6 000 und 9 000 nm mindestens 50 % der gesamten IR-Strahlung vorliegt.

11. Bestrahlungsgerät nach einem der Ansprüche 1 bis 10, dadurch gekennzeichnet, daß die Strahlungsquelle eine Hg-Niederdruck-Leuchtstofflampe (14, 41) umfaßt.

12. Bestrahlungsgerät nach Anspruch 11, dadurch gekennzeichnet, daß der Leuchtstoff Strontiumfluoroborat und Europium aufweist.

13. Bestrahlungsgerät nach einem der Ansprüche 1 bis 12, dadurch gekennzeichnet, daß die Strahlungsquelle (14) stabförmig und von einem rinnenförmigen Reflektor (15) umgeben ist.

14. Bestrahlungsgerät nach einem der Ansprüche 1 bis 13, dadurch gekennzeichnet, daß mehrere stabförmige Strahlungsquellen (14) dicht benachbart nebeneinander und mit geringem Abstand von der Strahlenaustrittsöffnung des Geräts (10, 20, 40) angeordnet sind.

15. Bestrahlungsgerät nach einem der Ansprüche 1 bis 14, dadurch gekennzeichnet, daß die in einer etwa horizontalen Ebene angeordneten Strahlungsquellen (14) von einer etwa horizontalen, zumindest für UV-Strahlung über 350 nm durchlässigen Liegeplatte (13) in geringem Abstand überdeckt sind.

16. Bestrahlungsgerät nach einem der Ansprüche 1 bis 14, dadurch gekennzeichnet, daß die in einer etwa horizontalen Ebene angeordneten Strahlungsquellen in einem Gehäuse (21) mit nach unten gerichteter Strahlenaustrittsöffnung untergebracht sind, wobei das Gehäuse mittels Motor (28) und zugehöriger Steuerung (29) auf und ab bewegbar ist.

## Claims

1. Medical radiation apparatus for treating cell tissue affected by carcinomatosis, characterised by a source of radiation the emission spectrum of which, i. e. energy distribution over wavelength, reaches a maximum in the UVA region, i. e. 315-400 nm, in which the emission spectrum maximum lies between 367 and 385 nm and the 50 % bandwidth of the emission spectrum is less than 30 nm, and a filter (4, 13, 16) which substantially filters out radiation under 340 nm.

2. Radiation apparatus according to claim 1, characterised in that the maximum lies between 370 and 380 nm.

3. Radiation apparatus according to one of the claims 1 or 2, characterised in that the maximum lies between 370 and 372 nm.

4. Radiation apparatus according to claim 1 to 3, characterised in that the emission spectrum falls off towards the short wave region to such a degree that the relative energy at 350 nm is less than 20 % of the maximum.

5. Radiation apparatus according to one of claims 1 to 4, characterised in that the filter (4, 13, 16) substantially filters out any radiation below 350 nm.

6. Radiation apparatus according to claim 5, characterised in that the 50 % bandwidth of the emission spectrum amounts to approximately 20 nm.

7. Radiation apparatus according to one of the claims 1 to 6, characterised in that the source of radiation (14) additionally emits IR-radiation in the region between 6 000 and 9 000 nm.

8. Radiation apparatus according to one of the claims 1 to 6, characterised in that an additional source of radiation (42) is provided which emits IR-radiation in the region between 6 000 and 9 000 nm.

9. Radiation apparatus according to claim 7 or 8, characterised in that the region between 6 000 and 9 000 nm contains at least 30 % of the total IR-radiation.

10. Radiation apparatus according to claim 9, characterised in that the region between 6 000 and 9 000 nm contains at least 50 % of the total IR-radiation.

11. Radiation apparatus according to one of the claims 1 to 10, characterised in that the source of radiation comprises a Hg-low pressure fluorescent lamp (14, 41).

12. Radiation apparatus according to claim 11, characterised in that the fluorescent material contains strontium fluoroborate and europium.

13. Radiation apparatus according to one of the claims 1 to 12, characterised in that the source of radiation (14) is rod-shaped and surrounded by a duct-shaped reflector (15).

14. Radiation apparatus according to one of the claims 1 to 13, characterised in that several rod-shaped sources of radiation (14) are arranged side by side in tight relationship and at a small distance from the radiation outlet opening of the apparatus (10, 20, 40).

15. Radiation apparatus according to one of the claims 1 to 14, characterised in that the sources of radiation (14) arranged approximately along a horizontal plane are covered at a small interval by an approximately horizontal plate (13) which is permeable at least to UV-radiation in excess of 350 nm.

16. Radiation apparatus according to one of the claims 1 to 14, characterised in that the sources of radiation arranged along an approximately horizontal plane are housed in a housing (21) having radiation outlet openings directed towards the bottom, whereby the housing can be moved up and down by means of a motor (28) and associated control means (29).

**Revendications**

1. Appareil d'irradiation médical destiné au traitement de tissus cellulaires présentant des modifications carcinomateuses, caractérisé par une source de rayonnement dont le spectre d'émission, c'est-à-dire la répartition de l'énergie suivant la longueur d'onde, présente un maximum dans la bande UVA, c'est-à-dire 315-400 nm, le maximum du spectre d'émission se situant entre 367 et 385 nm et la largeur de bande à 50 % du spectre d'émission n'étant pas supérieure à 30 nm, et par un filtre (4, 13, 16) qui élimine sensiblement le rayonnement inférieur à 340 nm.

2. Appareil d'irradiation selon la revendication 1, caractérisé en ce que le maximum se situe entre 370 et 380 nm.

3. Appareil d'irradiation selon l'une quelconque des revendications 1 ou 2, caractérisé en ce que le maximum se situe entre 370 et 372 nm.

4. Appareil d'irradiation selon la revendication 1 à 3, caractérisé en ce que le spectre d'émission présente en direction du domaine des faibles longueurs d'onde une chute suffisamment brusque pour qu'à 350 nm l'énergie relative soit inférieure à 20 % du maximum.

5. Appareil d'irradiation selon l'une quelconque des revendications 1 à 4, caractérisé en ce que le filtre (4, 13, 16) élimine sensiblement le rayonnement inférieur à 350 nm.

6. Appareil d'irradiation selon la revendication 5, caractérisé en ce que la largeur de bande à 50 % du spectre d'émission est d'environ 20 nm.

7. Appareil d'irradiation selon l'une quelconque des revendications 1 à 6, caractérisé en ce que la source de rayonnement (14) émet en outre un rayonnement IR dans le domaine entre 6 000 et 9 000 nm.

8. Appareil d'irradiation selon l'une quelconque des revendications 1 à 6, caractérisé en ce qu'il est prévu une source de rayonnement additionnelle (42) qui émet un rayonnement IR dans le domaine entre 6 000 et 9 000 nm.

9. Appareil d'irradiation selon la revendication 7 ou 8, caractérisé en ce que dans le domaine entre 6 000 et 9 000 nm se trouve au moins 30 % du rayonnement IR total.

10. Appareil d'irradiation selon la revendication 9, caractérisé en ce que dans le domaine entre 6 000 et 9 000 nm se trouve au moins 50 % du rayonnement IR total.

11. Appareil d'irradiation selon l'une quelconque des revendications 1 à 10, caractérisé en ce que la source de rayonnement comprend une lampe fluorescente à mercure sous basse pression (14, 41).

12. Appareil d'irradiation selon la revendication 11, caractérisé en ce que la matière fluorescente comporte du fluoroborate de strontium et de l'europium.

13. Appareil d'irradiation selon l'une quelconque des revendications 1 à 12, caractérisé en ce que la source de rayonnement (14) est en forme de barre et est entourée d'un réflecteur en forme de gouttière (15).

14. Appareil d'irradiation selon l'une quelconque des revendications 1 à 13, caractérisé en ce que plusieurs sources de rayonnement (14) en forme de barre sont juxtaposées tout près les unes des autres et sont disposées à une faible distance de l'ouverture de sortie de rayonnement de l'appareil (10, 20, 40).

15. Appareil d'irradiation selon l'une quelconque des revendications 1 à 14, caractérisé en ce que les sources de rayonnement (14) disposées dans un plan sensiblement horizontal sont recouvertes à une faible distance d'un plateau (13) sensiblement horizontal et perméable au moins au rayonnement UV supérieur à 350 nm.

16. Appareil d'irradiation selon l'une quelconque des revendications 1 à 14, caractérisé en ce que les sources de rayonnement disposées dans un plan sensiblement horizontal sont logées dans une enveloppe (21) présentant une ouverture de sortie de rayonnement dirigée vers le bas, l'enveloppe pouvant être montée et baissée au moyen d'un moteur (28) et d'une commande (29) associée à celui-ci.

## Fig.1

## Fig. 2

Fig. 3

Fig. 4

Fig. 6

Fig. 5